# EUROPÄISCHE PATENTSCHRIFT

(11) **EP 1 035 831 B1**
(45) Veröffentlichungstag und Bekanntmachung des Hinweises auf die Patenterteilung: **19.03.2003**
(21) Anmeldenummer: 98963545.3
(22) Anmeldetag: 08.12.1998
(51) Int. Cl.: A61K 7/48, A61K 33/00, A61K 33/10

(54) **VERWENDUNG VON KOHLENSÄURE ZUR STABILISIERUNG ODER ERHÖHUNG DER EPIDERMALEN CERAMIDSYNTHESERATE**
USE OF CARBOXYLIC ACID TO STABILIZE OR IMPROVE EPIDERMAL SYNTHESIZED CERAMIDE
UTILISATION DE GAZ CARBONIQUE POUR LA STABILISATION OU L'ELEVATION DE LA VITESSE DE SYNTHESE DE CERAMIDE EPIDERMIQUE

(30) Priorität: 09.12.1997 DE 19754659
(43) Veröffentlichungstag der Anmeldung: 20.09.2000
(73) Patentinhaber: Beiersdorf AG, 20253 Hamburg (DE)
(72) Erfinder: SCHWANITZ, Hans, Joachim, D-49076 Osnabrück (DE); BOCK, Meike, D-49082 Osnabrück (DE)
(74) Vertreter: Keller, Günter, Dr.
(86) Internationale Anmeldenummer: EP9807968
(87) Internationale Veröffentlichungsnummer: WO99029292

(56) Entgegenhaltungen:
- EP-A- 0 498 272
- PATENT ABSTRACTS OF JAPAN vol. 100, no. 78 & JP 60 215606 A (KAO SEKKEN), 29. Oktober 1985
- PATENT ABSTRACTS OF JAPAN vol. 111, no. 63 & JP 61 293908 A (KAO CO.), 24. Dezember 1986
- DATABASE WPI Week 8705 Derwent Publications Ltd., London, GB; AN 87-034000 XP002098778 & JP 61 291511 A (KAO CO.) , 22. Dezember 1986

## Beschreibung

Die vorliegende Erfindung betrifft die Verwendung von Kohlensäure zur Herstellung einer pharmazeutischen oder kosmetischen Zubereitung für die topische Applikation zur Stabilisierung oder Erhöhung der epidermalen Ceramidsyntheserate.

Die äußere Grenzschicht des Menschen zu seiner Umwelt ist die Haut. Eine der wichtigsten Aufgaben der Haut ist die Bildung einer Barriere zwischen äußerem und innerem Milieu. Diese Barriere, die zur Aufrechterhaltung eines konstanten inneren Milieus von entscheidender Bedeutung ist, wird als Permeabilitätsbarriere bezeichnet.

Zahlreiche Hauterkrankungen sind mit einer anlagebedingten oder erworbenen Störung der epidermalen Permeabilitätsbarriere assoziiert. Eine Stabilisierung bzw. der physiologische Aufbau der Permeabilitätsbarriere ist daher ein wichtiges Ziel der Externabehandlung und z.B. für Personen mit einer anlagebedingten empfindlichen Haut (atopischer Hautdiathese), für Personen mit trockener Haut und auch für die Prävention beruflich bedingter Hautveränderungen von entscheidender Bedeutung.

Obwohl die genaue Bewertung der Bedeutung aller einzelnen epidermalen Lipide für die Aufrechterhaltung der Barrierefunktion letztlich noch aussteht, existieren zur Bedeutung der Ceramide, einer Teilfraktion der epidermalen Lipide, für die Barrierefunktion zahlreiche Untersuchungen. So konnten zum Beispiel G. Imokawa et al. (Arch. Dermatol. Res. (1989) 281: 45-51) zeigen, daß die Wasserbindungsfunktion der Hornschicht und die Wasserpermeabilitätsbarrierefunktion der Haut in erster Linie durch die Lipidfraktion der Ceramide bestimmt werden.

Neue Befunde der Lipidforschung legen nahe, daß Defekte des epidermalen Lipidstoffwechsels und dabei insbesondere eine Verminderung der Ceramidmenge, zu Veränderungen des Zusammenhalts (Kohäsion) und der Abschuppung (Desquamation) der Hornzellen führen. Beeinträchtigungen der Barrierefunktion und Erkrankungen mit Verhornungsstörungen können die Folge sein (O. Braun-Falco, G. Plewig, H.H. Wolff, Dermatologie und Venerologie, 4. Auflage, 1996, Springer Verlag, Berlin Heidelberg New York).

Störungen im Lipidmuster bei Atopikern (Personen mit einer anlagebedingten erhöhten Hautempfindlichkeit, atopischer Dermatitis, Neurodermitis, endogenes Ekzem) konnten in verschiedenen Untersuchungen nachgewiesen werden. So konnten J. Hollmann et al. (H+G (1996) 71: 115-120) eine Verminderung des Ceramidgehalts der Hornschicht bei Neurodermitikern nachweisen.

In trockener Altershaut konnte eine verminderte Aktivität des Enzyms Sphingomyelinase nachgewiesen werden (T. Yamamura et al., J. Dermatol. Sci. (1990) 1: 79-84). Dieses Enzym ist in der Ceramidsynthese beteiligt und eine verminderte Aktivität führt zu einem Mangel an Ceramiden.

Diese Untersuchungen deuten auf eine zentrale Bedeutung epidermaler Lipide und insbesondere epidermaler Ceramide in der Pathogenese insbesondere der mit Sebostase (trockener Haut) und vermehrter Schuppenbildung einhergehenden Hauterkrankungen hin.

In der pharmazeutischen und kosmetischen Industrie hat man in den letzten Jahren aufgrund des zunehmenden Wissens zur Bedeutung der epidermalen Ceramide für die Barrierefunktion der Haut verschiedene Versuche unternommen, physiologische Ceramide in Kosmetika einzuarbeiten und so in die Haut einzuschleusen. Die Herstellung solcher Kosmetika ist jedoch wegen des technischen Aufwands sowie der Kosten für die Rohstoffe (Ceramide) wenig wirtschaftlich. Des weiteren ist es bis heute fraglich, ob ein Einbau der topisch aufgebrachten Ceramide in die epidermale Barriere überhaupt gelingt.

Darüber hinaus ist es bekannt, daß UV-A- und UV-B-Bestrahlungen die epidermale Ceramidsyntheserate erhöhen. Eine UV-Bestrahlung bei Hautveränderungen, die mit einer eingeschränkten Barrierefunktion assoziiert sind, zeigt gute therapeutische Effekte. In der Therapie des atopischen Ekzems kann diese Therapieform jedoch häufig nicht eingesetzt werden, da ein großer Teil von Atopikern lichtempfindlich ist. Des weiteren sind im Hinblick auf die Nebenwirkungen Risiken der UV-Bestrahlung bei der "Kosten-Nutzen"-Abwägung der Anwendung dieser Therapieform zu berücksichtigen.

Ein Verfahren zur therapeutischen Behandlung der Haut durch mit CO₂ imprägniertem Wasser ist aus der DE 41 24 728 bekannt. Hiermit sollen hartnäckige Hauterkrankungen und Durchblutungsstörungen behandelt werden. Eine Erhöhung der epidermalen Ceramidsyntheserate wird nicht offenbart.

Eine erhöhte Nutzbarmachung von Sauerstoff in der Haut durch eine topische CO₂-Anwendung wird auch von B. R. Hartmann et al. in Angiology 48 (4), 1997, Seiten 337-343 offenbart.

Eine verbesserte Durchblutung durch die topische Anwendung von CO₂ sowie die sich dadurch ergebende Anwendung zur Behandlung von Hautgeschwüren und -wunden wird von T. Ito et al. in J. Invest. Dermatol. 93 (2), 1989, Seiten 259-262 offenbart.

Bekannt ist somit die Wirkung von CO₂ auf periphere Durchblutungsstörungen sowie darauf zurückzuführende Erkrankungen, wie beispielsweise chronische Ulcera und Geschwüre.

Andere Hauterkrankungen wie die atopische Dermatitis sowie kumulativ-subtoxische oder traumiteraktive Ekzeme sind jedoch nicht auf Durchblutungsstörungen zurückzuführen, sondern unter anderem auf Störungen der Lipidsynthese, insbesondere der Ceramidsynthese. Es besteht somit ein Bedürfnis nach Zubereitungen, die die epidermale Ceramidsynthese stabilisieren oder erhöhen.

Es ist somit eine Aufgabe der vorliegenden Erfindung, eine Zubereitung zur Verfügung zu stellen, die die epidermale Ceramidsyntheserate stabilisiert oder erhöht.

Erfindungsgemäß wurde nun gefunden, daß die Applikation von Kohlensäure bzw. CO₂ die epidermale Ceramidsyntheserate stabilisiert oder erhöht.

Die Erfindung betrifft somit die Verwendung von Kohlensäure und/oder CO₂ zur Herstellung einer pharmazeutischen und/oder kosmetischen Zubereitung für die topische Applikation zur Stabilisierung oder Erhöhung der epidermalen Ceramidsyntheserate.

Die Stabilisierung oder Erhöhung der epidermalen Ceramidsyntheserate trägt zur Stabilisierung oder Verbesserung der Integrität der epidermalen Permeabilitätsbarriere und somit zur Verminderung des transepidermalen Wasserverlusts und zur Erhöhung der relativen Hautfeuchtigkeit bei. Hierdurch lassen sich beispielsweise atopische Xerosen, trockene Altershaut und irritative und kontaktallergische Ekzeme der Haut vorbeugen oder behandeln.

Allgemein können somit durch eine Stabilisierung oder Erhöhung der epidermalen Ceramidsyntheserate Hauterkrankungen durch eine Feuchtbelastung der Haut, wie beispielsweise ein kumulativ-subtoxisches oder traumiteraktives Ekzem, vorbeugend oder therapeutisch behandelt werden. Hierzu gehören berufsbedingte Hauterkrankungen, die zum überwiegenden Teil mit einer gestörten Permeabilitätsbarriere assoziiert sind. Darüber hinaus kann durch eine Stabilisierung oder Erhöhung der epidermalen Ceramidsyntheserate eine anlagebedingte erhöhte Hautempfindlichkeit (atopische Dermatitis, Neurodermitis, endogenes Ekzem) vorbeugend oder therapeutisch behandelt werden.

Für den Zweck der vorliegenden Erfindung wird unter einer Zubereitung, die als therapeutischen Wirkstoff Kohlensäure oder CO₂ enthält, jede Zubereitung verstanden, die unmittelbar Kohlensäure oder CO₂ enthält oder Kohlensäure oder CO₂ auf der Haut freisetzt. Hierbei kann es sich beispielsweise um wäßrige Zusammensetzungen handeln, die in Wasser gelöstes CO₂-Gas enthalten, wie im einfachsten Fall reines, kohlensäurehaltiges Wasser. Die Zubereitung kann beispielsweise auch als Lotion, Gel, Schaum, Creme, Wasch- oder Spülmittel vorliegen. Rezepturen für Zubereitungen, die Kohlensäure enthalten, finden sich beispielsweise unter Derwent-Ref.: 84-235086/78.

Ebenfalls geeignet sind Zusammensetzungen, die Kohlensäure erst bei Kontakt mit der Haut oder kurz vor der Anwendung freisetzen. Hierbei kann es sich beispielsweise um Mittel handeln, die auf die feuchte Haut aufgetragen werden, um im Kontakt mit Wasser in einer chemischen Reaktion Kohlensäure freizusetzen. Für eine Freisetzung von Kohlensäure kurz vor der Anwendung sind beispielsweise Badezusätze zu nennen. Diese Mittel, die Kohlensäure erst kurz vor oder bei der Anwendung des Medikaments freisetzen, können ein Carbonatsalz (Bicarbonat oder Hydrogencarbonat) wie Natrium- oder Kaliumcarbonat und eine organische Säure (z.B. Fruchtsäuren, wie Zitronensäure oder Weinsäure) enthalten, die in Gegenwart von Wasser Kohlensäure aus dem Carbonatsalz freisetzt. Bevorzugt enthalten diese Mittel auch ein Benetzungsmittel, damit die Reaktion zur Freisetzung der Kohlensäure möglichst rasch einsetzt, wenn das Mittel mit Wasser in Berührung kommt.

Die erfindungsgemäßen Zubereitungen können je nach Form und gewünschter Anwendungsart weitere übliche, pharmazeutisch verträgliche Zusätze enthalten.

Die wirksame Konzentration der Kohlensäure in der Zubereitung hängt von der Art und Dauer der Anwendung sowie der zu behandelnden Erkrankung ab und kann vom Fachmann leicht bestimmt werden. Der Gehalt an CO₂-Gas in kohlensäurehaltigem Wasser kann beispielsweise von 350 mg/l bis 1500 mg/l Wasser reichen. Der durch die Kohlensäure erzielte pH-Wert der Zubereitung kann zwischen 5 und 7, bevorzugt bei etwa 5,4 liegen.

Die vorliegende Erfindung stellt somit ein dermatologisches Therapeutikum zur Verfügung, das für eine Vielzahl von Patienten optimal sein kann. Optimal, da Kohlensäure als physiologischer Bestandteil des Organismus vorkommt und aufgrund seiner chemischen Eigenschaften nicht überdosiert werden kann. Unerwünschte Nebenwirkungen sind daher nicht zu erwarten.
- Fig. 1: zeigt die Mediane der Peakflächen für die Ceramidfraktion im Untersuchungsverlauf gemäß Beispiel 1.
- Fig. 2: zeigt die Mediane des transepidermalen Wasserverlusts (TEWL) im Untersuchungsverlauf gemäß Beispiel 2.

Die folgenden Beispiele sollen die vorliegende Erfindung weiter veranschaulichen.

### Beispiel 1

Dieses Beispiel veranschaulicht die Erhöhung der epidermalen Ceramidsyntheserate durch die Behandlung mit Kohlensäure.

### Methodik

Zum ersten Untersuchungstermin wurden auf jeweils 3 Teststellen am rechten und linken Unterarm Testpflaster mit 60 µl 1%igem Natriumlaurylsulfat (klassisches synthetisches Tensid, das in der dermatologischen Irritabilitätsforschung häufig angewendet wird) aufgeklebt und an der 4. Teststelle am rechten und linken Unterarm die epidermalen Lipide (Basiswerte vor Irritation) in vivo gewonnen. Nach 24 h wurden die Testpflaster entfernt und alle vorirritierten Teststellen erneut mit 1 % Natriumlaurylsulfat für 24 h okklusiv irritiert.

Durch die kumulative Irritation mit Natriumlaurylsulfat für 2 x 24 h an zwei aufeinanderfolgenden Tagen sollte zunächst eine Störung der epidermalen Barriere einschließlich einer Schädigung der epidermalen Lipidlamellen induziert werden.

An den folgenden Untersuchungstagen (Montag bis Freitag) wurden die Unterarme täglich mit Kohlensäure im Vergleich zu Süßwasser für jeweils 1 min gespült. Am ersten Untersuchungstag nach der Irritation (Montag) wurde vor der Spülung jeweils eine irritierte Teststelle an dem mit Kohlensäure und dem mit Süßwasser zu behandelnden Unterarm in vivo delipidiert. Nach 5- und 10tägiger Anwendung von Kohlensäure bzw. Süßwasser (Samstag) erfolgte die Delipidierung an jeweils einer der irritierten Teststellen am kohlensäure- bzw. süßwasserbehandelten Unterarm.

Die Anwendung von Süßwasser auf der Vergleichsseite erfolgte aus Gründen der Standardisierung. Das Süßwasser, als 1minütige Spülung angewendet, hat keine negativen Einflüsse auf die Permeabilitätsbarriere, dies konnte sowohl in eigenen Untersuchungen als auch von anderen Autoren gezeigt werden. Die Süßwasseranwendung auf der Vergleichsseite erlaubt somit die Aussage, daß gefundene Effekte ausschließlich auf die Kohlensäure zurückzuführen sind.

Das in vivo gewonnene Eluat aus Lösungsmittel und epidermalen Lipiden wurde zunächst unter Stickstoff evaporiert und die epidermalen Lipide anschließend eingefroren. Im Rahmen der Untersuchungen wurden so zunächst von allen Probanden die zu chromatographierenden Proben gesammelt.

Für die dünnschichtchromatographischen Untersuchungen wurden jeweils die 8 Proben eines Probanden aufgetaut und mit unterschiedlichen Konzentrationen des Standardgemisches auf einer Dünnschichtplatte entwickelt und detektiert.

Die Bestimmung des Lipidmusters vor Irritation, direkt nach Irritation nach 1wöchiger und nach 2wöchiger Anwendung von Kohlensäure bzw. Süßwasser erlaubt detaillierte Aussagen über die Regeneration der epidermalen Barriere und eventuelle Einflüsse der Kohlensäureanwendung auf einzelne Lipidfraktionen der epidermalen Barrierelipide.

Für die statistische Analyse wurde als parameterfreies Prüfverfahren der WILCOXON-Paardifferenzentest gewählt, da aufgrund der für die Basiswerte ermittelten Verteilungsdichtefunktionen die Hypothese der Normalverteilung zurückgewiesen werden mußte.

Als Signifikanzniveau für den Fall der Ablehnung der Nullhypothese wurde p < 0,01 (Irrtumswahrscheinlichkeit < 1 %) festgelegt.

### Ergebnisse

### Vergleichbarkeit der Testareale vor Beginn der Kohlensäurebehandlungen

Im Vergleich der Basiswerte für die Gesamtlipidmenge sowie für alle detektierten Lipidfraktionen zeigten sich im WILCOXON-Paardifferenzentest weder signifikante noch tendenzielle Unterschiede zwischen den Basiswerten für den mit Süßwasser und den mit Kohlensäure zu behandelnden Unterarm.

Nach der kumulativen Irritation mit Natriumlaurylsulfat zeigten sich weder für die delipidierte Gesamtlipidmenge noch für die einzelnen detektierten Lipidfraktionen in der statistischen Analyse signifikante bzw. tendenzielle Unterschiede zwischen dem mit Kohlensäure und dem mit Süßwasser zu behandelnden Testareal.

Hinsichtlich der Basiswerte und der nach Irritation ermittelten Werte für die Gesamtlipidmenge sowie für alle detektierten Lipidfraktionen ist somit eine Vergleichbarkeit der Testareale an dem mit Süßwasser und dem mit Kohlensäure zu behandelnden Unterarm gewährleistet.

### Ergebnisse für die Lipidfraktion der Ceramide

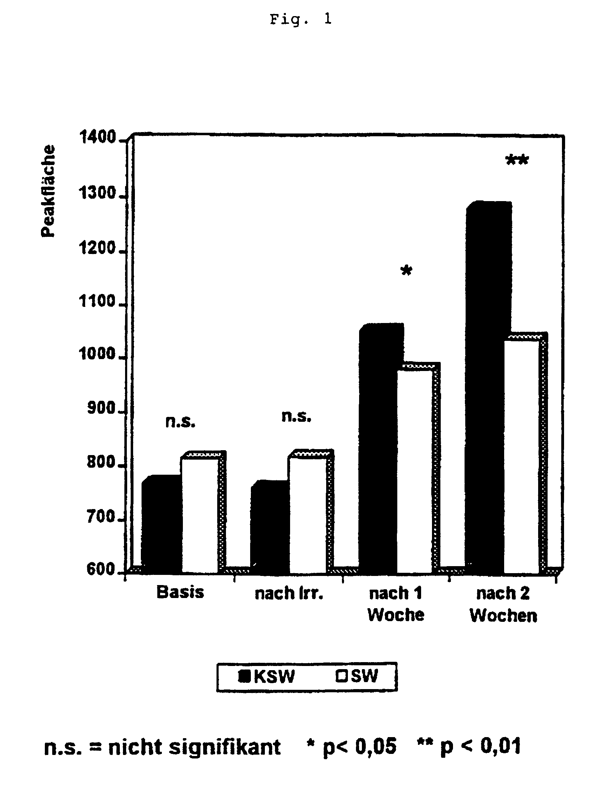

Figur 1 zeigt die Mediane der Peakflächen für die Ceramidfraktion zu den verschiedenen Untersuchungszeitpunkten. "KSW" bedeutet hierbei "Kohlensäurewasser", "SW" bedeutet "Süßwasser".

Für die Ceramidfraktion ließ sich in der statistischen Analyse im Vergleich des mit Kohlensäure und des mit Süßwasser behandelten Testareals kein Unterschied zwischen den Basiswerten und den Werten nach Irritation nachweisen.

Nach 1wöchiger Behandlung lagen jedoch die Werte für die Ceramidfraktion an dem mit Kohlensäure behandelten Testareal tendenziell (p < 0,05), nach 2wöchiger Behandlung signifikant (p < 0,01) höher als an dem mit Süßwasser behandelten Testareal.

Im Untersuchungsverlauf konnte sowohl an dem mit Kohlensäure als auch an dem mit Süßwasser behandelten Testareal nach einund zweiwöchiger Behandlung ein Anstieg der für die Ceramidfraktion ermittelten Peakflächen ermittelt werden. Während sich jedoch an dem mit Süßwasser behandelten Testareal die Werte für die Ceramidfraktion nach den Behandlungen nicht signifikant von den Basiswerten unterschieden, ließen sich an dem mit Kohlensäure behandelten Testareal im Vergleich zu den Basiswerten signifikant erhöhte Werte nachweisen.

Die durch Natriumlaurylsulfat induzierte epidermale Barriereschädigung resultiert in einem Anstieg der epidermalen Ceramidsynthese. Durch die Kohlensäurebehandlung läßt sich eine signifikante Steigerung der Ceramidpeakflächen im Vergleich zur Süßwasserbehandlung erreichen, was als deutlich günstiger Einfluß der Kohlensäureanwendung auf die epidermale Ceramidsynthese nach Barriereschädigung zu werten ist.

### Beispiel 2

Dieses Beispiel zeigt die Verminderung des transepidermalen Wasserverlusts (TEWL) und die Erhöhung der relativen Hautfeuchtigkeit (RHF) und damit die Verbesserung der epidermalen Permeabilitätsbarriere durch eine Behandlung mit Kohlensäure.

### Materialien

Für die standardisierte Waschung wurde ein normales im Handel erhältliches Shampoo, das nach Angaben des Herstellers 19 % aktive Reinigungssubstanzen enthält, mit destilliertem Wasser verdünnt, bis es 10 % aktive Reinigungssubstanzen in der gebrauchsfertigen Lösung enthielt.

Für die Süßwasserteilgüsse wurde Leitungswasser verwendet. Der pH des Süßwassers war während des gesamten Testzeitraums bei 8,2 ± 0,1 relativ konstant.

Das eingesetzte kohlensäurehaltige Wasser wurde mit Hilfe einer Imprägnierungsvorrichtung mechanisch hergestellt, in der Leitungswasser unter Überdruck mit Kohlendioxid angereichert wurde. Der pH des kohlensäurehaltigen Wassers (pH 5,4) war während der gesamten Dauer der Untersuchung konstant.

Alle Teilgüsse erfolgen bei einer Temperatur von 37°C.

### Methodik

Der Test wurde als Halbseitenversuch an 20 Testpersonen (13 Frauen und 7 Männer im Alter zwischen 23 - 28 Jahren) durchgeführt. Die freiwilligen Testpersonen wurden keinen Auswahlbedingungen unterworfen, außer der Rechtshändigkeit. Zur zufälligen Verteilung wurde eine Hälfte der Teilnehmer mit Kohlensäure auf der rechten Hand behandelt und die andere Hälfte wurde auf der linken Hand behandelt.

Über einen Zeitraum von 2 Wochen wurde ein Handrücken der Testpersonen einmal täglich für 1 min mit Kohlensäure gespült. Der andere wurde als Kontrolle mit Süßwasser gespült. Gleichzeitig wurden die Rücken beider Hände wiederholt mittels einer standardisierten Waschung mit der Shampoolösung zweimal täglich irritiert.

Der Einfluß von Kohlensäure oder Süßwasser auf die hautphysiologischen Parameter irritierter Haut wurde unter Verwendung nichtinvasiver hautphysiologischer Untersuchungsmethoden gemessen.

Der transepidermale Wasserverlust (TEWL) als ein Maß der Permeabilitätsbarrierefunktion wurde unter Verwendung der Evapometriemethode gemessen. Ein Evaporimeter EP1 (Servomed & Co., Hässelby, Stockholm, Schweden) wurde verwendet. Die relative Hautfeuchtigkeit (RHF) wurde unter Verwendung der Kapazitätsmessungsmethode gemessen. Ein Corneometer CM 820 PC (Courage & Khazaka Electronic GmbH, Köln, Deutschland) wurde verwendet. Die Messungen wurden als der Mittelwert von 2 Aufnahmen (Evapometrie) oder 3 Aufnahmen (Corneometrie) angegeben.

Alle Tests fanden im teilklimatisierten Hautphysiologielabor der Universität Osnabrück statt. Aufgrund der nur geringfügigen Schwankungen bezüglich der Temperatur (20°C ± 1°C) und der Luftfeuchtigkeit (48 % ± 4 %) konnten schwankende Korrelationen zwischen Temperatur/Luftfeuchtigkeit und den zu erfassenden hautphysiologischen Parametern ausgeschlossen werden.

Den Untersuchungen war eine 30minütige Akklimatisationsphase vorgeschaltet, um eine Anpassung der Probanden an die definierten Laborbedingungen zu gewährleisten. Während der Akklimatisationsphase wurde eine Testfläche mit einem Durchmesser von 1,5 cm auf den Handrücken der Freiwilligen nahe der mittleren metacarpo-phalangealen Verbindung markiert. Nach der Akklimatisationsphase wurden die TEWL- und RHF-Basiswerte in allen Testbereichen gemessen. Sofort danach fand die erste Waschung statt. Im Anschluß daran wurden die Handrücken mit Kohlensäure bzw. Süßwasser gespült. Die zweiten Messungen wurden 30 min nach der Applikation durchgeführt, da Wasser, sowohl beim Benetzen als auch Abspülen, einen zeitweiligen aber trotzdem beachtlichen Anstieg in der Feuchtigkeit des Stratum Corneums verursacht. Schließlich fand die zweite Waschung in gleicher Art und Weise wie die erste statt.

### Statistische Auswertung

Der Median und der 25/75-Prozentwert wurden als Standard für die Statistik gewählt. Der WILCOXON-Paardifferenztest wurde für die parameterfreie Statistik verwendet. Er gilt als verteilungsfreies Analog zum t-Test für gepaarte Meßwerte und ist als Symmetrietest um den Median der Differenzen zu verstehen (L. Sachs, Angewandte Statistik, 6. Auflage, Berlin, Springer-Verlag, 1984).

Neben dem Verhältnis der positiven und negativen Differenzen, das Aussagen über die Zusammenhänge zwischen zwei Parametern möglich macht, wurde das folgende Signifikanzniveau gewählt: P < 0,05.

### Ergebnisse

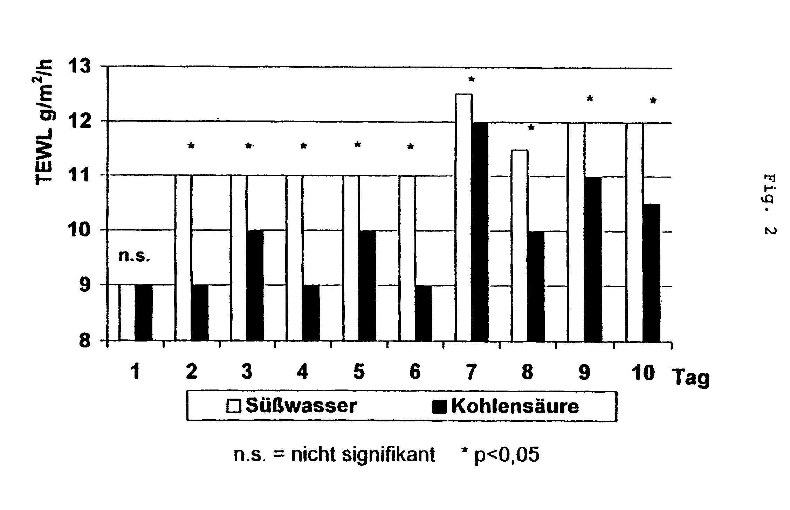

Die durch die wiederholten Waschungen verursachte Irritation führte zu einem signifikanten Anstieg der TEWL-Werte, sowohl in Kombination mit den Süßwasser- als auch in Kombination mit den Kohlensäureteilgüssen. Der Vergleich der Medianwerte zeigte jedoch einen geringeren Anstieg des TEWL in den mit Kohlensäure behandelten Testarealen. Der sich aus dem geringeren Anstieg ergebende Unterschied wurde im WILCOXON-Paardifferenztest deutlich: An den Tagen 3, 6, 7 und 8 waren die TEWL-Werte signifikant niedriger an dem mit Kohlensäure behandelten Testareal als an dem mit Süßwasser behandelten. Dieser positive Einfluß der topisch applizierten Kohlensäure auf den TEWL irritierter Haut wurde beim Vergleich der Werte 30 min nach Anwendung noch klarer. Mit Ausnahme des ersten Tags war der TEWL der mit Kohlensäure behandelten Testregionen 30 min nach Anwendung signifikant niedriger als in den mit Süßwasser behandelten Testregionen. Diese Ergebnisse sind in Fig. 2 wiedergegeben, die die TEWL-Mediane jeweils 30 min nach Anwendung von Kohlensäure bzw. Süßwasser im Untersuchungsverlauf zeigen.

Die Basiswerte der RHF und der RHF 30 min nach Anwendung waren in den mit Kohlensäure behandelten Testgebieten höher (in einigen Fällen signifikant höher) als in den mit Süßwasser behandelten.

Dieses Ergebnis zeigt, daß eine kontrollierte Verbesserung der Permeabilitätsbarriere, deren Funktion direkt mit dem TEWL korreliert, durch die topische Applikation von Kohlensäure erreicht werden kann. Wenn man bedenkt, daß eine Vielzahl von Hauterkrankungen, wie beispielsweise die atopische Xerose, die trockene Altershaut und irritative sowie kontaktallergische Ekzeme mit einer Störung der Permeabilitätsbarriere in Verbindung gebracht werden, wird die Bedeutung einer kontrollierten Verbesserung der Permeabilitätsbarriere klar. Eine solche Verbesserung wird durch die vorliegende Erfindung erreicht.

## Patentansprüche

1. Verwendung von Kohlensäure und/oder CO₂ zur Herstellung einer pharmazeutischen Zubereitung für die topische Applikation zur Prävention oder Behandlung einer Erkrankung durch Stabilisierung oder Erhöhung der epidermalen Ceramidsyntheserate.

2. Verwendung nach Anspruch 1, worin die Zubereitung zur Stabilisierung oder Verbesserung der epidermalen Permeabilitätsbarriere dient.

3. Verwendung nach Anspruch 2, worin die Zubereitung den transepidermalen Wasserverlust vermindert.

4. Verwendung nach Anspruch 2 oder 3, worin die Zubereitung die relative Hautfeuchtigkeit erhöht.

5. Verwendung nach einem der vorhergehenden Ansprüche, worin die Zubereitung zur Prävention oder Behandlung von Hauterkrankungen durch Feuchtbelastung dient.

6. Verwendung nach Anspruch 1, worin die Zubereitung zur Prävention oder Behandlung der atopischen Dermatitis dient.

7. Kosmetische Verwendung von Kohlensäure und/oder CO₂ zur Stabilisierung oder Erhöhung der epidermalen Ceramidsyntheserate, wobei die Kohlensäure und/oder das CO₂ in einer kosmetischen Zubereitung für die topische Applikation vorliegt.

8. Verwendung nach Anspruch 7 zur Stabilisierung oder Verbesserung der epidermalen Permeabilitätsbarriere.

9. verwendung nach Anspruch 8 zur Verminderung des transepidermalen Wasserverlustes.

10. Verwendung nach Anspruch 8 oder 9 zur Erhöhung der relativen Hautfeuchtigkeit.

## Claims

1. The use of carbonic acid and/or CO₂ for producing a pharmaceutical preparation for topical application for the prevention or treatment of a disorder by stabilizing or increasing the epidermal ceramide synthesis rate.

2. The use as claimed in claim 1, wherein the preparation is used for stabilizing or improving the epidermal permeability barrier.

3. The use as claimed in claim 2, wherein the preparation reduces the transepidermal water loss.

4. The use as claimed in claim 2 or 3, wherein the preparation increases the relative skin moistness.

5. The use as claimed in any of the preceding claims, wherein the preparation is used to prevent or treat skin disorders due to moisture stress.

6. The use as claimed in claim 1, wherein the preparation is used to prevent or treat atopic dermatitis.

7. Cosmetic use of carbonic acid and/or CO₂ for stabilizing or increasing the epidermal ceramide synthesis rate, wherein the carbonic acid and/or CO₂ are in the form of a cosmetic preparation for topical application.

8. The use as claimed in claim 7 for stabilizing or improving the epidermal permeability barrier.

9. The use as claimed in claim 8 for reducing the transepidermal water loss.

10. The use as claimed in claim 8 or 9 for increasing the relative skin moistness.

## Revendications

1. Utilisation d'acide carbonique et / ou de CO₂ pour la production d'une préparation pharmaceutique pour application locale en vue de la prévention ou du traitement d'une affection par stabilisation ou augmentation de la vitesse de synthèse des céramides épidermiques.

2. Utilisation selon la revendication 1, où la préparation sert à la stabilisation ou à l'amélioration de la barrière de perméabilité épidermique.

3. Utilisation selon la revendication 2, où la préparation réduit la déperdition d'eau transépidermique.

4. Utilisation selon la revendication 2 ou 3, où la préparation augmente l'humidité relative de la peau.

5. Utilisation selon l'une des revendications qui précèdent, où la préparation sert à la prévention ou au traitement de maladies de peau par hydratation.

6. Utilisation selon la revendication 1, où la préparation sert à la prévention ou au traitement de la dermatite atopique.

7. Utilisation cosmétique d'acide carbonique et / ou de CO₂ pour la stabilisation ou l'augmentation de la vitesse de synthèse des céramides épidermiques, où l'acide carbonique et / ou le CO₂ sont présents dans une préparation cosmétique pour application locale.

8. Utilisation selon la revendication 7 pour la stabilisation ou l'amélioration de la barrière de perméabilité épidermique.

9. Utilisation selon la revendication 8 pour la réduction de la déperdition d'eau transépidermique.

10. Utilisation selon la revendication 8 ou 9 pour l'augmentation de l'humidité relative de la peau.
